Europäisches Patentamt

(19) European Patent Office

Office européen des brevets

(11) **EP 0 611 307 B1**

(12) **EUROPEAN PATENT SPECIFICATION**

(45) Date of publication and mention
of the grant of the patent:
**28.05.1997 Bulletin 1997/22**

(21) Application number: **92922009.3**

(22) Date of filing: **14.10.1992**

(51) Int. Cl.6: **A61L 2/14**

(86) International application number:
**PCT/EP92/02379**

(87) International publication number:
**WO 93/07908 (29.04.1993 Gazette 1993/11)**

(54) **METHOD FOR TREATING A SURFACE USING A PLASMA STREAM**

VERFAHREN ZUR BEHANDLUNG EINER OBERFLÄCHE MITTELS EINES PLASMASTROMES

PROCEDE DE TRAITEMENT DE SURFACES A L'AIDE D'UN FLUX DE PLASMA

(84) Designated Contracting States:
**AT BE CH DE DK ES FR GB IT LI NL SE**

(30) Priority: **15.10.1991 SU 5006406**

(43) Date of publication of application:
**24.08.1994 Bulletin 1994/34**

(73) Proprietor: **OPA (OVERSEAS PUBLISHERS
ASSOCIATION) AMSTERDAM, B.V.
1075 Amsterdam (NL)**

(72) Inventors:
• **KULIK, Pavel P.**
  **Zelenograd Moscow, 103498 (RU)**
• **IVANOV, Vladimir V.**
  **Zelenograd Moscow, 103498 (RU)**
• **ZORINA, Eugenia N.**
  **Zelenograd Moscow, 103498 (RU)**
• **TSVETKOVA, Yulia V.**
  **Zelenograd Moscow, 103498 (RU)**

(74) Representative: **Orchard, Oliver John
  JOHN ORCHARD & CO.
  Staple Inn Buildings North
  High Holborn
  London WC1V 7PZ (GB)**

(56) References cited:
**FR-A- 2 654 000**

**Description**

This invention relates to the treatment of surfaces, and, in particular, although not exclusively, it has application in the treatment of the surfaces of medical equipment. The invention may, for example, be used for the sterilization and/or the disinfection of both medical instruments and accessories.

Known in the art is a method for the sterilization and the disinfection of medical equipment which includes the step of treating the equipment at a high-temperature in special chambers (at 200° C); see V.I. Vashkov. "Equipment and Methods for Sterilization in Medicine" (in Russian). M., M. 1973, pp. 271; 269-297.

It is also known to sterilize and disinfect the surfaces of medical equipment by acting upon the surface physically under atmospheric pressure; see M.A. Tumanyan, D.A. Kaushansky. "Radiation Sterilization". M., M. 1974. pp. 139-165.

It should be noted that the above prior art methods are characterised by the need for a long treatment time (up to several hours) and that they cannot ensure a high quality of sterilization and disinfection. The use of a method according to the invention results in both a substantial decrease in treatment time (to fractions of a second) and a high quality of treatment.

These results are accomplished, in one particular method, by acting upon a surface with a stream of nitrogen and/or argon plasma and by causing there to be relative movement between the surface to be treated and a zone of the plasma stream acting upon the surface, the relative movement taking place at least once at a speed between 0.6 and 1.5 m/s.

A method according to the invention which will now be described, by way of example, is carried out in the following manner. A surface of medical equipment to be treated is subjected to a dynamic plasma treatment with an incoming relatively stationary plasma stream having an enthalpy of braking $1>10$ J/kg under non-stationary heating conditions provided by causing the surface being treated to pass through the plasma stream with $T_{i,a} = T_s < T_a$, wherein $T_{i,a}$ are the temperatures of ion and neutral components of the plasma stream and the surface being treated respectively; $T_s$ is the temperature of the surface being treated; $T_a$, is the admissible temperature of heating of the surface being treated; USSR Inventor's Certificate No. 1457732, H 01 L 21/306, 1987. The treatment is carried out with a stream of nitrogen and/or argon plasma. The surface being treated is caused to pass through a zone of the plasma stream at least once at a speed of 0.6 to 1.5 m/s.

As a result of controlling the relative speed between the surface and the plasma stream acting upon the surface, and the number of times the surface is exposed to the plasma stream, a mode can be chosen which results in the surface of medical equipment being sterilized and disinfected without damage to the equipment.

The method will now be illustrated, in particular, by way of examples 1 and 2.

Microbiological material was prepared and treated at the All-Union Institute of Prophylactic Toxicology and Disinfection of the USSR Ministry of Health. Plasma treatment was carried out at the Engineering Center "Plazmodinamika". Plasma was generated by means of two and four-jet arc generators. Argon or nitrogen were used as plasma forming gas. Enthalpy of braking of the plasma stream was $10^7$ to $10^9$ J/kg.

The results of sterilization and disinfection using the dynamic plasma treatment method described are given in Tables 1 and 2.

Example 1

B. Subtilis and Bac. cereus were used as test cultures for sterilizing. The design number of microorganisms applied to test objects subjected to sterilization was $10^3$ microbial bodies per 1 cm$^2$. The test results are given in Table 1.

Table 1

| Materials | Treatment speed, m/s | Number of passes, n | Efficiency | | |
|---|---|---|---|---|---|
| | | | Used | Sterilized | % |
| Glass | 0.6 | 1 | 11 | 11 | 100 |
| | 0.6 | 2 | 2 | 2 | 100 |
| | 0.6 | 5 | 9 | 9 | 100 |
| | 0.6 | 10 | 9 | 9 | 100 |
| Glass | 1.0 | 1 | 11 | 11 | 100 |
| | 1.0 | 5 | 14 | 14 | 100 |
| Glass | 1.5 | 1 | 23 | 21 | 92 |
| | 1.5 | 3 | 13 | 11 | 87 |
| | 1.5 | 5 | 10 | 9 | 90 |
| Silicon plates | 0.6 | 1 | 21 | 21 | 100 |
| | 0.6 | 2 | 4 | 4 | 100 |
| | 0.6 | 3 | 9 | 9 | 100 |
| Silicon plates | 1.0 | 1 | 9 | 9 | 100 |
| | 1.0 | 3 | 9 | 9 | 100 |
| Silicon plates | 1.5 | 1 | 10 | 8 | 80 |
| | 1.5 | 2 | 2 | 2 | 100 |
| | 1.5 | 3 | 14 | 14 | 100 |

The level to which the cultures on both the glass and the silicon plates were killed was 100% when passed through the plasma stream at a speed of 0.6 to 1.0 m/s. With a speed of 1.5 m/s the efficiency of sterilization during a period of 0.2 s. was found to be dependent upon the type of material.

Example 2

Sterile test objects of glass and silicon plate of a size of 1cm$^2$ were seeded with a suspension of golden staphylococcus, or B. cereus, or coliphase fd52 (2 billion of microbial bodies per 1 ml) so that $10^7$ of said bodies were present in the tests. Sterile samples were carried in Petri dishes to a test site and were treated in various modes. The samples were then transferred to a laboratory in sterile test tubes and were placed in a culture media.
The results are given in Table 2.
It can be seen from Table 2 that the tests were carried out in two-jet and four-jet modes. That is plasma streams from two jets and four jets were used respectively. The following legends are used in Table 2:

EP 0 611 307 B1

TABLE 2

| Microorganism | Dynamic plasma treatment mode | Treatment speed, m/s | Material | Number of passes | | | | Control without dynamic plasma treatment |
|---|---|---|---|---|---|---|---|---|
| | | | | 3 | 5 | 10 | 20 | |
| Coliphage | double-jet | 0.6 | glass | 1- | | | | |
| | | 1.0 | glass | 1- | 1- | 3- | 3- | + |
| | | 1.5 | silicon | 1+ | 3- | 3- | 3- | + |
| | | | glass | 2- | 1- | | | |
| Golden Staphylococcus | " | 1.0 | glass | 3- | 3- | | | + |
| | " | | | | | | | |
| B. cereus | " | 1.0 | glass | 3- | | | | + |
| B. cereus | four-jet | 1.0 | glass | 3- | | | | + |
| | | 1.5 | glass | 1- | | | | + |
| | | | silicon | 2- | | | | + |

1- - no culture growth on the test material;
1+ - growth of culture on the test material;
3- - no culture growth in three tests.

The test results attest to the fact that vegetative forms of bacteria (golden staphylococcus) and bacteriophage (coliphage f52) did not survive in a steady manner with a speed of movement of the support through a plasma stream of from 0.6 to 1.5 m/s in three passes. It will be apparent from Table 2 that sporiferous organisms were killed with the two-jet mode on glass at a speed of movement of the plate surface through a plasma stream of 1 m/s in 20 passes during 1.0 s and in 10 passes in 0.5 s.

Therefore, it will be seen that with the methods of dynamic plasma treatment described it is possible to achieve high-speed and complete sterilization and disinfection of the surfaces of equipment, such as medical equipment, using any of the above-mentioned modes, and that the range of products which may be treated is greater than with previously known treatment methods.

It will also be appreciated that, although in the preferred embodiments described the position of the plasma stream does not move relative to the object being treated, because the object is moved relative to the stream, it is possible, in certain situations, to move the plasma stream relative to the object being treated.

Particular embodiments of the invention have been described, by way of example only, and it will be appreciated that variations and modifications thereof, as well as other embodiments, may be made within the scope of the appended claims.

**Claims**

1. A method for use in the treatment of a surface which is suitable for the purpose of sterilization and/or disinfection, in which the surface is acted upon by means providing a physical treatment, characterised in that the treatment includes the steps of exposing the surface to a stream of nitrogen and/or argon plasma, and causing relative movement between the surface to be treated and a zone of the plasma stream as it acts upon the surface, the relative movement taking place at a speed between 0.6 and 1.5 m/s.

2. A method as claimed in claim 1 in which the relative movement is between the surface and a plurality of plasma streams.

3. A method as claimed in any one of the preceding claims in which the surface is moved and the plasma stream or streams is/are maintained relatively stationary.

4. A method as claimed in any one of the preceding claims in which the relative movement takes place more than once.

4

**Patentansprüche**

1. Verfahren zur Verwendung bei der Behandlung einer Oberfläche, das für Sterilisations- und/oder Desinfektionszwecke geeignet ist, bei dem mittels Mitteln, die eine physikalische Behandlung vornehmen, auf die Oberfläche eingewirkt wird, dadurch gekennzeichnet, dass die Behandlung die Schritte des einem Strahl von Stickstoff- und/oder Argon-Plasma Aussetzens der Oberfläche und des Erzeugens einer relativen Bewegung zwischen der zu behandelnden Oberfläche und einem Bereich des Plasmastrahls, so wie er auf die Oberfläche einwirkt, umfasst, wobei die relative Bewegung mit einer Geschwindigkeit zwischen 0,6 und 1,5 m/s stattfindet.

2. Verfahren nach Anspruch 1, bei dem die relative Bewegung zwischen der Oberflache und einer Mehrzahl von Plasmastrahlen stattfindet.

3. Verfahren nach einem der vorhergehenden Ansprüche, bei dem die Oberfläche bewegt wird und der Plasmastrahl oder die Plasmastrahlen relativ stationär gehalten wird/werden.

4. Verfahren nach einem der vorhergehenden Ansprüche, bei dem die relative Bewegung mehr als einmal stattfindet.

**Revendications**

1. Méthode pour l'utilisation dans le traitement d'une surface, qui est convenable pour la stérilisation et/ou la désinfection, dans laquelle on opère sur la surface par des moyens effectuant un traitement physique, caractérisée en ce que le traitement comprends les pas d'exposer la surface à un jet de plasma d'azote et/ou d'argon et de causer un mouvement relatif entre la surface à traiter et une zone du jet de plasma telle qu'elle opère sur la surface, le mouvement relatif ayant lieu à une vitesse entre 0,6 et 1,5 m/s.

2. Méthode selon la revendication 1, dans laquelle le mouvement relatif a lieu entre la surface et une pluralité de jets de plasma.

3. Méthode selon l'une des revendications précédentes, dans laquelle la surface est bougée et le jet de plasma ou les jets de plasma est/sont maintenu(s) relativement stationnaire.

4. Méthode selon l'une des revendications précédentes, dans laquelle le mouvement relatif a lieu plus qu'une fois.